# EUROPEAN PATENT APPLICATION

(11) **EP 1 724 265 A1**
(43) Date of publication of application: **22.11.2006**
(21) Application number: 04772348.1
(22) Date of filing: 27.08.2004
(51) Int. Cl.: C07D 257/10, A61K 31/395, A61P 35/00

(54) **ANTICANCER AGENT**

(30) Priority: 08.03.2004 JP 2004063370
(71) Applicant: Ono, Nobufumi, Jounan-ku, Fukuoka-shi Fukuoka 8140153 (JP)
(72) Inventor: Ono, Nobufumi, Jounan-ku, Fukuoka-shi Fukuoka 8140153 (JP)
(74) Representative: Robertson, James Alexander
(86) International application number: PCT/JP2004/012392
(87) International publication number: WO 2005/085215

(57) **Abstract**

An anticancer agent according to the present invention is difficult for a cancer cell to excrete and is suitable for topical treatment of cancer cells. The anticancer agent according to the present invention contains as an active ingredient the catenane compound indicated by the chemical formula (I) below. 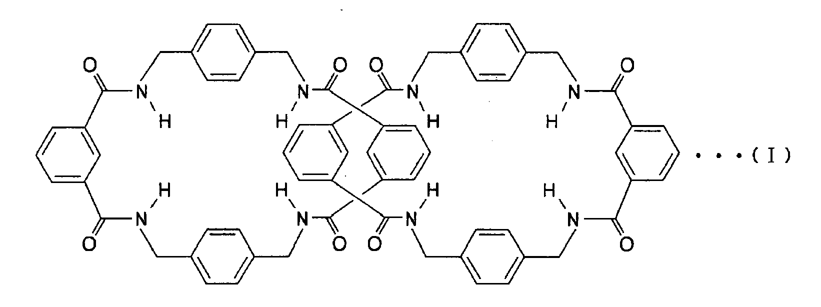 This catenane compound is a type of compound referred to as amide [2] catenane, and has a structure in which two molecular rings linked to each other like a chain are not bonded to each other by covalent bonding. The shape of the molecule can be easily changed and the molecule is relatively difficult to be incorporated via a receptor, but the proliferation of cancer cells is inhibited when this compound is introduced into cancer cells.

## Description

### TECHNICAL FIELD

The present invention relates to an anticancer agent, and particularly relates to an anticancer agent containing a catenane compound as an active ingredient thereof.

### BACKGROUND OF THE INVENTION

The average lifespan in Japan has increased by ten or more years compared to thirty years ago, and a major reason for this increase is considered to be the development of numerous drugs for treating stroke and heart disease, which have been the leading causes of death. However, as stroke and heart disease decline as leading causes of death, deaths due to cancers (malignant neoplasms) increase each year. Cancer is currently the leading cause of death in Japan, and development of anticancer agents is therefore being pursued by a large number of researchers.

Because most anticancer agents work only after being absorbed into a cancer cell, drugs having low molecular weights and stable structures that can be easily incorporated via a receptor have been considered effective in the past.

### SUMMARY OF THE INVENTION

However, anticancer agents that are easily absorbed via a receptor are also often easily excreted from the cell, and therefore suffer from the drawback of poor retention in the cancer cell. Once a cancer cell has excreted an anticancer agent, the cell becomes resistant to that anticancer agent, and the anticancer agent becomes ineffective.

Because anticancer agents that are easily absorbed via a receptor are also easily incorporated into normal cells, topical treatment is difficult and side effects easily occur.

Therefore, an object of the present invention is to provide a novel anticancer agent that is difficult for a cancer cell to excrete.

Another object of the present invention is to provide a novel anticancer agent that is suitable for topical treatment of cancer cells.

The inventor focused attention on unique polymer compound devoid of a covalently bonded moiety (see Japanese Patent Laid Open No. H11-80136), such as catenane or rotaxane, as a compound not easily excreted from a cancer cell. This type of unique polymer compound has a relatively large molecular weight and readily deformable molecules. This polymer is therefore considered to be difficult to excrete once it has been incorporated into the cancer cell. Since this type of unique polymer compound is naturally difficult for a cell to absorb, it becomes possible to dramatically reduce the effect of this compound on normal cells if the compound can be topically applied to cancer cells.

As a result of concentrated investigation from this perspective, the inventor discovered that a certain catenane compound has anticancer effects.

Specifically, the anticancer agent according to the present invention is characterized in comprising as an active ingredient a catenane compound indicated by the chemical formula (I) below.

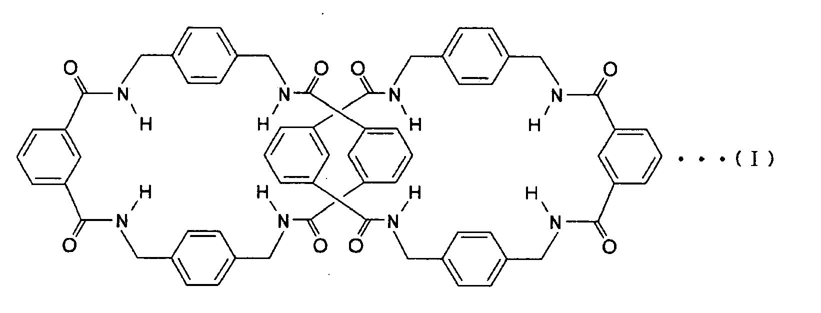

This catenane compound is a type of compound referred to as an amide-type [2]catenane, and is formally referred to as 3,11,18,26-tetraazapentacyclo [26.2.2.213,16.15,9.120,24]hexatriaconta-5,7,9(36),13, 15,20,22,24(33),28,30,34-dodecaene-4,10,19,25-tetrone (9C1). As indicated by the chemical formula (I) above, this catenane compound has a structure in which two molecular rings are linked like a chain, and these two molecular rings are not bonded to each other by covalent bonding. The molecule therefore changes shape easily, and incorporation of the molecule via a receptor becomes relatively difficult, but it was learned that once this compound is introduced into a cancer cell, the proliferation of cancer cells is inhibited.

The preferred method for introducing this catenane compound into a cancer cell involves making a hole in the cell membrane of the cancer cell and introducing the compound into the cell through this hole. Electroporation may be used as the method for creating a hole in the cell membrane. Since the abovementioned catenane compound can be locally introduced when electroporation is used, the quantity in which the compound is incorporated into normal cells can be kept extremely low. As a result, it becomes possible to alleviate side effects and other strains on the patient.

At present, the catenane compounds for which anticancer effects have been verified by the inventor include only the catenane compound indicated by the chemical formula (I) above. However, considering that catenane compounds in general are characteristically difficult to incorporate into a cell, and are not easily excreted once incorporated into the cell, catenane compounds having anticancer effects other than the catenane compound indicated by the abovementioned chemical formula (I) are predicted to exist. Rotaxane compounds having anticancer effects are also predicted to exist.

### BRIEF DESCRIPTION OF THE DRAWINGS

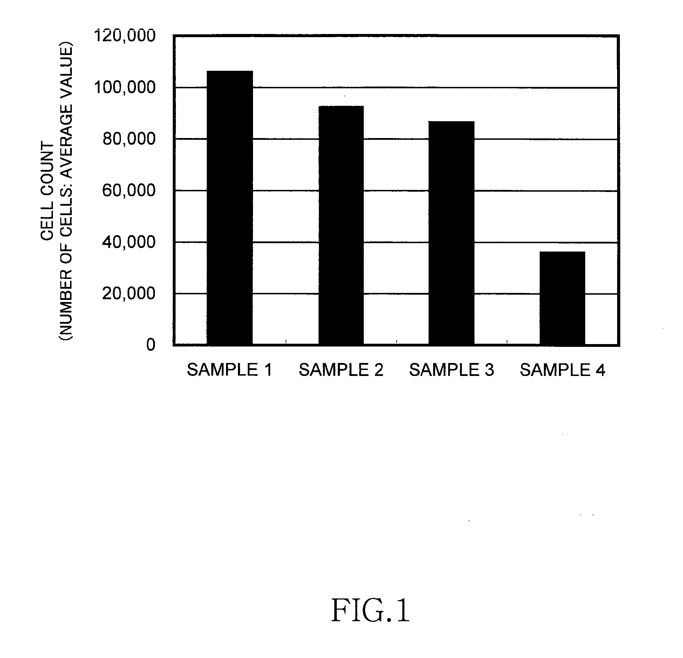

FIG. 1 is a graph showing the evaluation results for samples 1 through 4.

### DETAILED DESCRIPTION OF THE EMBODIMENTS

Preferred embodiments of the present invention will now be described hereinafter.

The anticancer agent according to the present invention contains as an active ingredient a catenane compound indicated by the chemical formula (I) below. 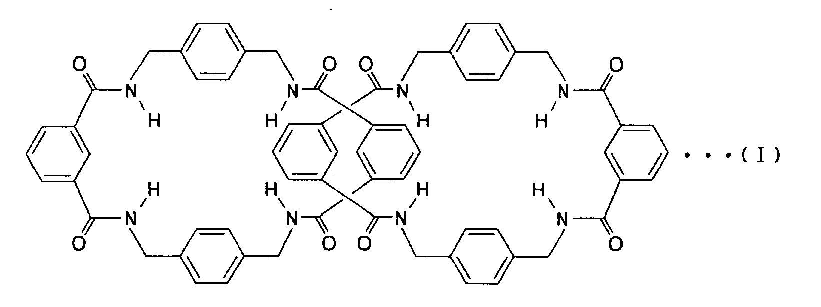 The method for synthesizing this catenane compound is not particularly limited, but this compound can be synthesized by reacting isophthaloyl dichloride and p-xylylene diamine.

The catenane compound obtained in this manner is soluble in dimethyl sulfoxide (DMSO) and other solvents, and can therefore be used in solution as an anticancer agent. The catenane concentration in the solution is not particularly limited, but is preferably 300 µg/mL or greater. This is because although inhibiting effects on proliferation of cancer cells are obtained even when the catenane concentration in the solution is less than 300 µg/mL, these effects become prominent when the concentration is 300 µg/mL or greater.

The two molecular rings forming the catenane compound are not covalently bonded, and therefore easily change shape and are relatively difficult to incorporate via a receptor. Therefore, it is preferred that a hole be opened in the cell membrane of the cancer cell using an electroporation method in order to introduce this catenane compound into the cancer cell. The voltage waveform in this case is preferably that of a DC pulse, the pulse voltage is preferably about 100 V, and the pulse period is preferably about 10 msec.

Since the cancer cell cannot easily excrete this catenane compound once it is incorporated into the cancer cell by electroporation or another method, not only can the catenane compound be utilized over a long period of time, but resistance to the catenane compound is extremely difficult to develop.

### EXAMPLES

Examples of the present invention will next be described.

### [Cell Sampling]

First, mouse colon-26 cells (colon cancer cells) were attached to the inner wall of a culture flask.

The culture flask was then set so that the surface to which the cancer cells were attached was on the bottom, a medium (RPMI, 8 mL) not containing blood serum was caused to flow into the culture flask, the cancer cells were cultured successively for approximately one week, and the cells were brought into a state of sufficient adhesion (confluent state). The medium in the culture flask was then discarded, and the inside of the culture flask was further rinsed with a medium (RPMI, 5 mL) not containing blood serum. Nearly all of the cells not adhering to the inner wall of the culture flask were thereby discarded, and only those cells which proliferated while attached to the inner wall remained.

A trypsin-containing solution of EDTA (ethylene diamine tetraacetate) in the amount of 5 mL was then placed in the culture flask, and the cells deposited on the inner wall were separated from the wall, after which the entire contents of the flask were transferred to a culture tube. The inside of the culture flask was also rinsed with a medium (RPMI, 5 mL) containing blood serum in order to neutralize the trypsin, and the entire contents of the flask were transferred to a culture tube.

This culture tube was then centrifuged (1000 rpm, 5 minutes), after which the supernatant fluid was discarded, and a suspension was formed in a medium (RPMI, 10 mL) containing blood serum. A liquid suspension was thereby obtained as a bulk sample.

### [Electroporation operation]

An 800-µL sample (sample 1) composed of 720 µL of the abovementioned suspension and 80 µL of DMSO was created, and the sample was placed in a cuvette having a pair of electrodes.

Another 800-µL sample (sample 2) was prepared from 720 µL of the abovementioned suspension and 80 µL of a DMSO solution having a concentration of 60 µg/mL of the catenane indicated by chemical formula (I), and this sample was placed in a separate cuvette.

Another 800-µL sample (sample 3) was prepared from 720 µL of the abovementioned suspension and 80 µL of a DMSO solution having a concentration of 105 µg/mL of the catenane indicated by chemical formula (I), and this sample was placed in another cuvette.

Another 800-µL sample (sample 4) was prepared from 720 µL of the abovementioned suspension and 80 µL of a DMSO solution having a concentration of 300 µg/mL of the catenane indicated by chemical formula (I), and this sample was placed in another cuvette.

A DC pulse (pulse voltage: 104 to 108 V; pulse period: 9 to 11 msec) was then applied to the electrodes of the cuvettes containing the samples 1 through 4, and electroporation was performed.

After electroporation was completed, the samples 1 through 4 were each transferred to 15-mL culture tubes having a serum-containing medium (RPMI, 5 mL), and the contents of the culture tubes were centrifuged (1000 rpm, 5 minutes), after which the supernatant fluid was discarded, and a suspension was formed in a medium (RPMI, 3 mL) containing blood serum.

### [Sample evaluation]

After rinsing with a medium (RPMI, 500 µL) not containing blood serum, four sets of 24 wells were prepared containing a serum-containing medium (RPMI, 500 µL), 40 µL each of the samples 1 through 4 for which electroporation was performed were transferred to the corresponding 24 wells, and the cells were cultured for three days.

After culturing, the cells in each well were stained by a trypan blue staining method, and the cell count was taken under a microscope. The results thereof are shown in FIG. 1. As shown in FIG. 1, the cell count after culturing was inversely proportional to the catenane concentration, and inhibiting effects on cancer cell proliferation were thereby confirmed for the catenane indicated by the chemical formula (I). Particularly in the sample 4 in which the catenane concentration was 300 µg/mL, the cell count was reduced to 1/3 that of the catenane-free sample 1, and significant inhibiting effects on cancer cell proliferation were confirmed.

### [Toxicity evaluation]

The catenane indicated by the chemical formula (I) was suspended in an aqueous solution of 0.25% CMC (carboxymethylcellulose), and a 20 mg/kg dose thereof was orally administered to approximately 25-g male mice (two mice). Both mice still survived after one week of observation, and no abnormal behavior was identified during the observation period. The mice also ingested about the same amount of food and water as normal mice. It was thereby confirmed that the catenane indicated by the chemical formula (I) has adequately low toxicity.

## Claims

1. An anticancer agent containing as an active ingredient a catenane compound indicated by the chemical formula (I) below.

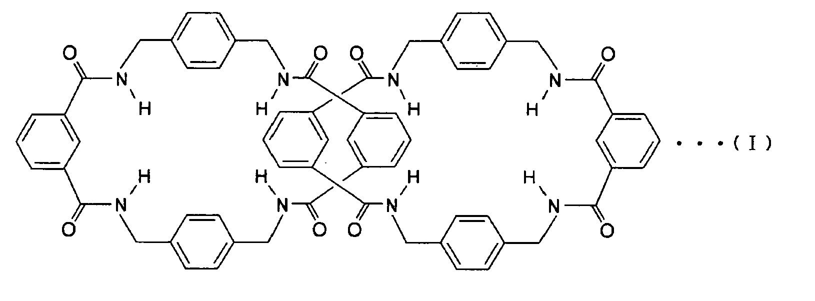

2. The anticancer agent as claimed in claim 1, wherein said catenane compound is dissolved in a solution, and the concentration of said catenane compound in said solution is 300 µg/mL or greater.
